(19) Europäisches Patentamt
European Patent Office
Office européen des brevets

(11) EP 4 095 129 A1

(12) EUROPEAN PATENT APPLICATION
published in accordance with Art. 153(4) EPC

(43) Date of publication:
30.11.2022 Bulletin 2022/48

(21) Application number: 20913884.1

(22) Date of filing: 17.01.2020

(51) International Patent Classification (IPC):
*C07D 235/06* (2006.01) *C07D 403/06* (2006.01)
*C07D 409/06* (2006.01) *C07D 471/04* (2006.01)
*C07D 495/04* (2006.01) *C07J 43/00* (2006.01)
*A61K 31/4184* (2006.01) *A61P 35/00* (2006.01)
*A61P 43/00* (2006.01)

(52) Cooperative Patent Classification (CPC):
A61K 31/4184; A61P 35/00; A61P 43/00;
C07D 235/06; C07D 403/06; C07D 409/06;
C07D 471/04; C07D 495/04; C07J 43/00

(86) International application number:
PCT/RU2020/000019

(87) International publication number:
WO 2021/145785 (22.07.2021 Gazette 2021/29)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: Farber, Boris Slavinovich
Moscow 121151 (RU)

(72) Inventors:
- MARTYNOV, Artur Viktorovich
Kharkov, 61171 (UA)
- MERZLIKIN, Serhii Ivanovych
Kharkov, 61121 (UA)
- FARBER, Boris Slavinovich
Moscow, 121151 (RU)

(74) Representative: McQueen, Andrew Peter
Miltons IP - Europe
5th Floor, Broad Quay House
Prince Street
Bristol BS1 4DJ (GB)

# (54) BENZIMIDAZOLE DERIVATIVES AND SALTS THEREOF EXHIBITING AN ANTI-GERIATRIC EFFECT

(57) Various benzimidazole derivatives based on thermoleabile compounds, for example, amino acids and vitamins, exhibiting geroprotective activity in the experiment and can be used in the development of agents for prolonging the life of humans and animals, are proposed.



Processed by Luminess, 75001 PARIS (FR)

## Description

### Technology area

The invention part of organic pharmaceutical chemistry, it can be used in pharmacy, medicine and cosmetology for the prevention and / or treatment of diseases associated with aging process.

### Prior art

The emergence of selective agonists of $I_1$-imidazoline receptor has led to the class of centrally acting antihypertensive drugs (sympatholytics) in the treatment of arterial hypertension (AH). At the same time, the fact of the return of sympatholytics "second birth" to cardiological practice after a long break was officially recorded. Drugs in this group were among the first antihypertensive drugs that began to be used in clinical practice about 40 years ago, since in the pathogenesis of hypertension, the sympathetic nervous system was of great importance since the time of the neurogenic theory of G.F. Lang.

However, when it became clear that older generation sympatholytics (clonidine, methyldopa, reserpine) often cause serious side effects as drowsiness, depression, sexual dysfunction and the rebound phenomenon, they were no longer widely used as drugs for long-term antihypertensive therapy. They were used either for hypertensive crises or for economic reasons due to their relatively low cost. At the same time, the understanding of the importance of the sympathetic nervous system in the genesis of hypertension was so rooted in the consciousness of the medical community that research attempts to create new, effective, and safe sympatholytics has not stopped.

The relevance of the creation of such drugs increased even more when it became clear that the activation of the sympathetic nervous system leads to an increase in blood pressure (BP) and plays a role in the occurrence of a number of other negative effects, including metabolic disorders, which significantly increase the risk of complications in persons with hypertension. Among these effects, suffice it to mention myocardial hypertrophy, endothelial dysfunction, platelet activation, insulin resistance, and dyslipidemia. Thus, one of the most important areas of pharmacotherapy for hypertension is a multifaceted approach that decreases activity of the sympathoadrenal system, corrects metabolic disorders and assists in organoprotection.

With the discovery of imidazoline receptors and the creation of its selective agonists, the emergence of new effective and safe sympatholytics has become a reality. French scientists have established that imidazoline receptors are located in the two most important organs of blood pressure regulation - the brain and kidneys.

They are located in the lateral reticular nuclei of the rostral region of the medulla oblongata and in the proximal tubules of the kidneys. It turned out that these structures do not react to catecholamines, but react to chemical compounds similar to imidazoline. That is why these receptors were named imidazoline receptors. Activation of these receptors at the level of the brain causes modulation of sympathetic impulses and a decrease in blood pressure, and effect in the kidneys, a decrease in the activity of the H + / Na + pump and slowing down the reabsorption of salt and water.

### Imidazoline receptor agonists

Agonists of imidazoline receptors (AIR), having a structure similar to imidazoline, bind to these receptors in the brain and a. By acting on the imidazoline receptors in the brain, they reduce sympathetic activity. This results in a decrease in peripheral vascular resistance, the activity of the renin-angiotensin system and the reabsorption of salt and water. On the other hand, due to its high affinity for imidazoline receptors, AIR in the therapeutic doses practically does not affect other adrenergic receptors, such as, $\alpha 2$ receptors. Not affecting other adrenergic receptors allows AIR to cause side effects less often compare to other centrally acting drugs.

As it known, the appearance of these side effects is associated with the stimulation of $\alpha 2$-adrenergic receptors, through which both selective (a-methyldopa) and non-selective (clonidine) $\alpha 2$-adrenergic receptor agonists exercise their antihypertensive effect. The data of a several studies indicate the antihypertensive efficacy of agonists of $I_1$-imidazoline receptor, comparable to the efficacy of the most well-known and widely used representatives of the main classes of antihypertensive drugs. AIR do not have a "slip away" effect or develop a tolerance for treatment. On the other hand, AIRs are well tolerated due to the fact, as mentioned above, that in therapeutic doses they do not affect other adrenergic receptors. That is why the analysis of data on the metabolic effects of AIR is of particular interest.

The most convincing results were obtained in the studies of Haenni A. et al. using the euglycemic clamp test method *[*Haenni, A., & Lithell, H. (1994). Treatment with a $\beta$-blocker with $\beta 2$-agonism improves glucose and lipid metabolism in essential hypertension. Metabolism, 43(4), 455-461.]. It has been found that moxonidine reduces insulin resistance. This study was conducted at the State Research Center for Preventive Medicine of Russian Federation, which included patients with mild to moderate hypertension and compensated diabetes mellitus type 2. The results showcased a positive effect of moxonidine on insulin resistance. After 3 months of treatment with moxonidine, there

was a significant decrease in insulin and blood glucose levels determined 2 hours after a standard breakfast (equivalent to a glucose tolerance test). These results indicate an improvement in tissue insulin sensitivity, due to less insulin required to maintain therapeutic glucose levels after moxonidine therapy.

In a comparative randomized study ALMAZ, which involved 202 patients with confirmed insulin resistance, the study was conducted on effect of moxonidine and metformin on glucose metabolism in patients with hypertension and with obesity [*https://htn.almazovcentre.ru/jour/article/view/33?locale=en_US#*]. The study showed that moxonidine reduced fasting glucose levels, insulin resistance, patient weight, and also increased the rate of glucose utilization. The effect of these drugs on the glycemic profile in patients who are overweight, have mild hypertension, insulin resistance or impaired glucose tolerance was also evaluated. With moxonidine, fasting glucose decreased less pronounced than with metformin, but serum insulin levels decreased significantly, while the decrease in body mass index was comparable with the use of both drugs. Interesting data was obtained in a study of the simultaneous effect of imidazoline receptor agonists on sympathetic activity and metabolic parameters. The study, focused on the efficacy of moxonidine, and included 41 patients with grade 1-3 hypertension (grade III risk).

In order to assess the activity of moxonidine, all patients before and after treatment of the sympathoadrenal system, in addition to the standard study of blood biochemical parameters (glycemic level, HbA1c, blood lipid spectrum), underwent a double dynamic test (DDT) for catecholamines (dopamine, norepinephrine, adrenaline). Also, leptin levels were examined in all patients. After 8 weeks of treatment with moxonidine, the achievement of the target BP level was accompanied by a significant decrease in the level of stress hormones, body mass index, insulin resistance and leptin concentration. It should be noted that during treatment, a shift in the blood lipid spectrum towards antiatherogenicity was recorded - a significant decrease in triglycerides and an increase in high-density lipoprotein cholesterol (HDL). Thus, the drug Moxogamma reproduces not only the antihypertensive, but also the favorable metabolic effects of the original drug, which, of course, positions it as a modern high-quality generic drug.

**Influence on endothelial function**

The ability of this group of drugs to improve endothelial function should have considered to be of significant clinical importance. Endothelial dysfunction is currently considered as a universal mechanism for the implementation of the atherogenic influence of various risk factors. Correction of endothelial dysfunction in addition to antihypertensive action can provide an effective reduction in the risk of cardiovascular complications in long-term therapy of hypertension. One of the indicators allowing to assess the function of the endothelium is the fibrinolytic activity of blood plasma. As you know, normal fibrinolytic activity is provided by a balance between levels tissue plasminogen activator (tPA) and its inhibitor (PAI-1), which are synthesized in endothelial cells. An increase in PAI-1 synthesis leads to a decrease in fibrinolytic activity, increasing the risk of progression of cardiovascular diseases. A significant decrease in the level of PAI-1 was found during therapy with moxonidine in patients with hypertension, one of the possible mechanisms of which is a decrease in insulin resistance and the activity of the sympathoadrenal system [Krespi PG, Makris TK, Hatzizacharias AN, et al. Moxonidine effect on microalbuminuria, thrombomodulin, and plasminogen activator inhibitor - 1 levels in patients with essential hypertension. J Cardiovasc Drugs and Therapy 1998; 12: 463-467.].

There was also a decrease in the plasma level of thrombomodulin, a glycoprotein of membranes of endothelial cells, which is playing role as a receptor for thrombin and appears in blood plasma when the endothelium is damaged. Therefore, the decrease in thrombomodulin during therapy with moxonidine is probably associated with the maintenance of the integrity of the vascular endothelium. Thus, the results of recent studies have shown that selective agonists of $I_1$-imidazoline receptors provide not only adequate and long-term control of blood pressure, but also have a number of positive metabolic effects: a decrease in insulin resistance, increase in HDL cholesterol, improvement in endothelial function and improvement fibrinolytic activity of blood plasma. In the European guidelines of diagnosis and treatment of hypertension, AIR is assigned to the best class of drugs in terms of a beneficial effect on tissue sensitivity to insulin [2007 Guidelines for the Management of Arterial Hypertension: The Task Force for the Management of Arterial Hypertension of the European Society of Hypertension (ESH) and of the European Society of Cardiology (ESC). J Hypertens 2007;25(6):1105-87.]. In the Russian guidelines for the diagnosis and treatment of hypertension, the AIR niche is designated as the treatment of hypertension in combination with metabolic syndrome in combination with ACE inhibitors or angiotensin II receptor blockers. It is emphasized that these combinations not only efficaciously reduce blood pressure, but also have a beneficial effect on target organs and reduce the risk of developing diabetes mellitus. Thus, the positive metabolic effects and organoprotection of AIR have received official recognition.

**Agmatine as a high affinity ligand for imidazoline receptors**

The hypothesis of the existence of imidazoline receptors was put forward by a group of researchers who studied the central hypotensive effect of the I2-adrenergic receptor agonist clonidine, which has an imidazoline group in its structure. Subsequently, in experiments on neurons in the rostral ventrolateral zone of the medulla oblongata (RVLM)

of rats, evidence was obtained that at least 36% of specific binding sites in this zone differ from adrenergic ones and recognize imidazoline derivatives. Based on experiments with radioligands of different selectivity, the main types of imidazoline receptors were identified. I1, -receptors are marked with [3H] -clonidine and "recognize" all imidazoline and imidazole compounds, as well as oxazoline derivatives. I2, - receptors have a high affinity for imidazoline derivatives (clonidine, moxonidine), medium affinity for imidazole derivatives (idazoxane, phentolamine) and low affinity for guanidine derivatives (amiloride, guanabenz). I, -receptors are involved in the implementation of the central antihypertensive effect of clonidine.

I2 receptors are labeled with [3H] -idazokean and recognize some imidazolines, benzodiazepines, and guanidine compounds. I2 receptors have a high affinity for imidazole and guanidine compounds, medium affinity for imidazole and new compounds. I2 receptors are divided into 2 subtypes: I2a - with high and I2b - with low affinity for amiloride. Recently, the classification of imidazoline receptors has been supplemented with I3 receptors.

They have been found in the pancreas. Imidazoline receptors of various types are localized in the central and peripheral nervous system, as well as in the heart, kidneys, stomach, pancreas, liver, large intestine, placenta, and prostate gland. These receptors are involved in the reactions of the cardiovascular system, the regulation of intraocular pressure, the control of the secretion of hydrochloric acid in the stomach, the release of insulin, and the modulation of nociceptive responses. Imidazoline receptors have also been studied in connection with their possible participation in the development of pathological aging processes, such as depression, Alzheimer’s disease, Parkinson’s disease, and glial tumors.

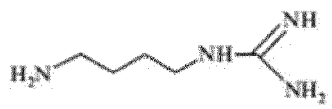

The existence of imidazoline binding sites as a separate type of receptor suggested the presence of their endogenous ligand. In recent years, three main candidates for this role have been identified from animal tissues: the classical clonidine-substituted substance (cCDS), the immunoreactive clonidine-substituted substance (iCDS), and agmatine.

Of the three candidates, the structure of the ligand of imidazoline receptors was determined only in agmatine. Agmatine, isolated from the mammalian brain in 1994, is decarboxylated arginine:

Agmatine binds to imidazoline receptors of all subtypes in the concentration range of 0.5-5 mM. It is impossible to assert at present that agmatine is a selective endogenous ligand of imidazoline receptors due to its relatively low affinity and insufficient data on the effects of agmatine associated with action on these receptors.

**Terminology**

**Antigeriatric action** - pharmacological effects aimed at prolonging the life of a living organism: these drugs include histone deacetylase inhibitors, phosphodiesterase inhibitors, type I1 and I2 imidazoline receptor stimulants, activators of telomerase expression, immunomodulators, antiviral agents, anticancer agents, statins, and antihyperglycemic drugs (metformin). A common generic feature of this pharmacological action is the physical extension of human or animal life.

A common generic feature of this pharmacological action is the physical extension of human or animal life.

**Telomerase** is an enzyme that adds specific repeating DNA sequences (TTAGGG in vertebrates) to the 3’-end of the DNA strand in telomere regions that are located at the ends of chromosomes in eukaryotic cells. Telomeres contain condensed DNA and stabilize chromosomes. With each cell division, the telomeric regions are shortened. The existence of a mechanism that compensates for the shortening of telomeres (telomerase) was predicted in 1973 by A.M. Olovnikov. Telomerase is a reverse transcriptase, and a special RNA molecule is associated with it, which is used as a template for reverse transcription during telomere elongation.

As a **result of telomerase activity,** the length of telomeric regions of the cell’s chromosomes increases or remains at a constant level, thus compensating for terminal under replication and allowing the cell to divide for an unlimited time. In the course of the study of this enzyme (consisting, as described below, of the RNA component and the protein component), it was found that the RNA component is expressed at a constant level in almost all cells, and the expression of the protein component is required to induce telomerase activity, which is therefore called the catalytic component of telomerase.

**Artificially induced expression of the gene** for the catalytic component of telomerase (by introducing the gene using genetic engineering methods) makes the cell culture immortal, that is, capable of dividing indefinitely, thereby canceling the Hayflick limit for culture. Telomerase is expressed in stem, sex, and some other types of cells in the body, which must constantly divide for the functioning of certain tissues (for example, intestinal epithelial cells). Ordinary somatic cells of the body are devoid of telomerase activity. Cells of 85% of cancerous tumors have telomerase activity;

therefore, telomerase activation is considered to be one of the activator factor of the cell to malignant transformation.

**Known** compounds with activity against imidazoline receptors (Application WO2000002878A1), which are three-membered cycles - carbolines, containing a benzimidazole fragment. The synthesized substances are supposed to be used as antihypertensive agents, neuroprotectors, nephro- and cardioprotectors. The authors showed more than 100-fold affinity for I2-receptors (according to the results of modeling) in benzodioxan, than for adrenergic receptors. It has been shown the accumulation of certain substances in the brain of rabbits. -The disadvantages of these compounds are the absence of geroprotective action (prolongation of life span). These compounds have a higher complexity of preparation, and lack reliable pharmacological data on biological activity compared to the one that was proposed by us.

## Disclosure of invention

**The objective of the invention** is to obtain new derivatives of benzimidazole, their salts, compositions exhibiting antigeriatric action.

The task is solved by synthesizing new benzimidazole derivatives of the following structure:

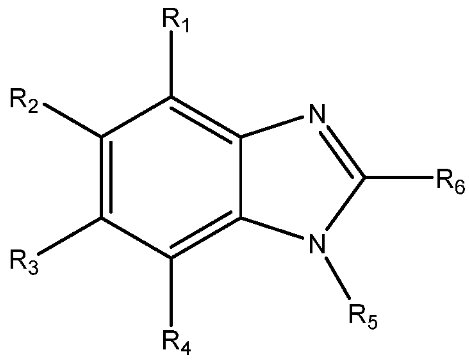

ГДе $R_{1-4}$ = H, $-CH_3$, $-C_2H_5$, -Br, - Cl, -I, -F, -OH, -CN, $-NO_2$, -COOH.

$R_5$= H, $-CH_3$, $-C_2H_5$.

**The R6 substituent can have one of the following structures:**

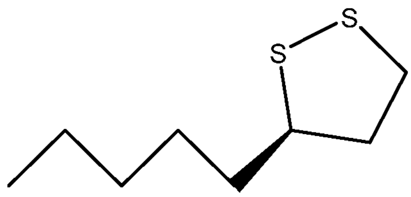

;

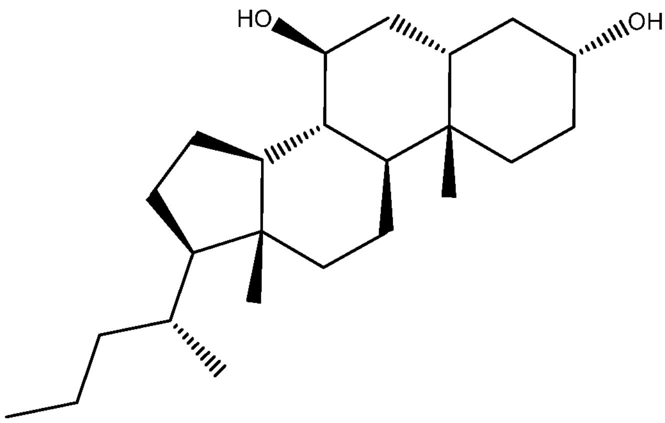

;

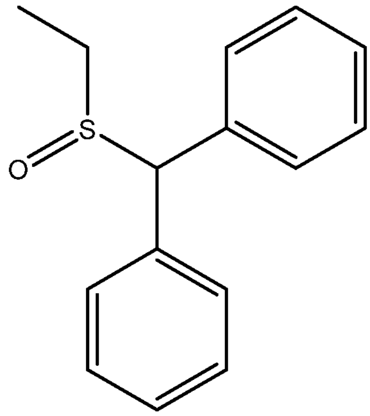

;

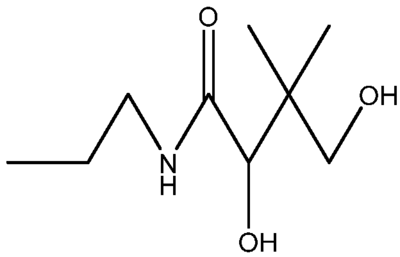

;

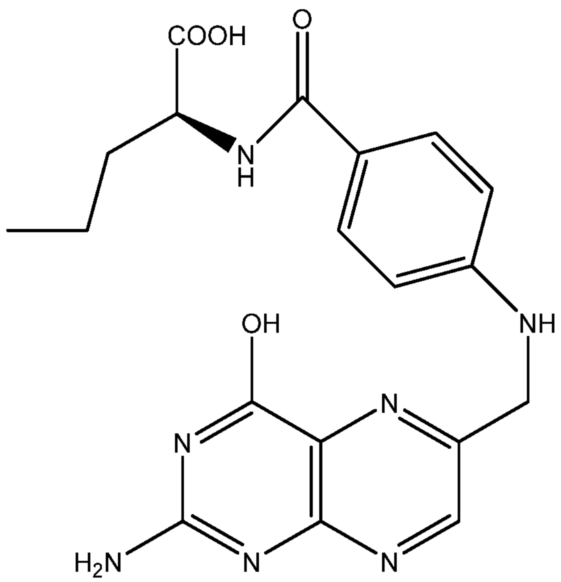

;

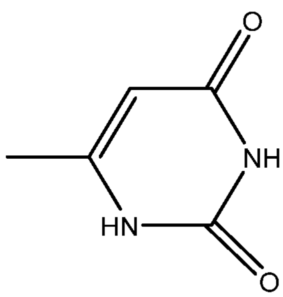

;

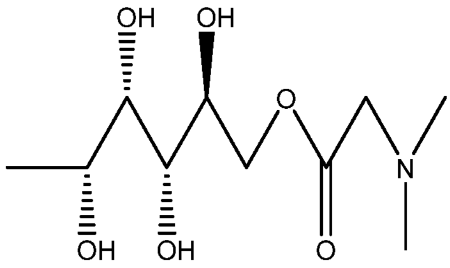
;

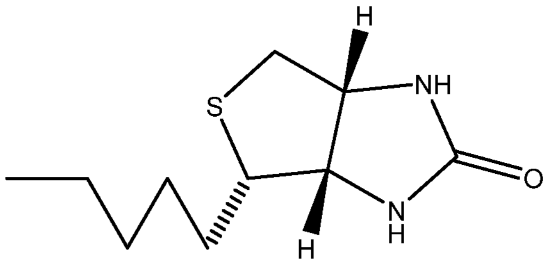
;

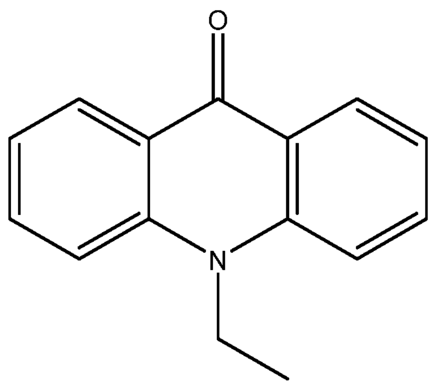

Also

$R_6$= 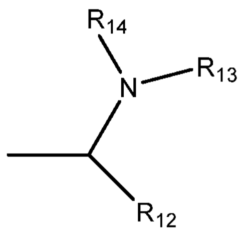

$R_{13,14}$= -H; - $CH_3$, -$C_2H_5$
$R_{12}$= - $CH_3$

$R_{12}$= 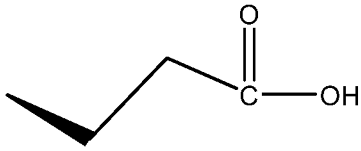 ;

$R_{12}$= 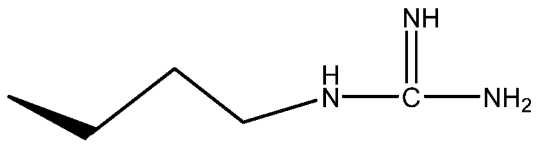 ;

$R_{12}$= 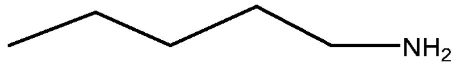 ;

$R_{12}$= 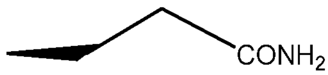 ;

$R_{12}$= 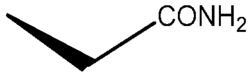 ;

$R_{12}$= 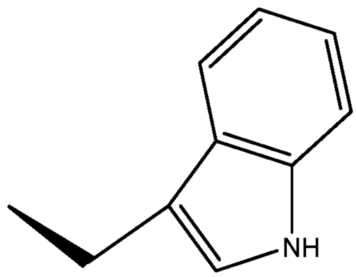 ;

$R_{12}$= 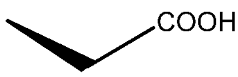

The above structures can be salts: hydrochlorides, hydrobromides, hydroiodides, sodium, potassium, magnesium, calcium, iron, copper salts. They can also be salts with thioctic acid, metformin, choline, dapagliflozin and a complex with 3- (1H-benzimidazol-2-yl) -1,2,2-trimethylcyclopentanecarboxylic acid.

These complexes and salts can be used in the manufacture of drugs - selective activators of imidazoline receptors for the treatment of pathologies caused by aging. Based on these structures, pharmaceutical compositions for the treatment of pathologies caused by aging of the body can be obtained, containing an effective amount of the above derivatives from 0.5 mg to 1000 mg in combination with one or more pharmaceutically acceptable excipients.

Separately, the composition may contain, as a mixture, thioctic acid, metformin, choline dapagliflozin, 3- (1H-benzimidazol-2-yl) -1,2,2-trimethylcyclopentanecarboxylic acid alone or in mixture with each other. This pharmaceutical composition can be used to obtain different dosage forms as injection solutions or suspensions, incl. in multi-dose vials, plain or coated tablets, sugar-coated tablets, plate capsules, gel capsules, pills, cachets, powders, suppositories or rectal capsules, solutions or suspensions, topical ointments and gels.

The pharmaceutical compositions developed and described above can be used for parenteral, oral, rectal, permucous, or transdermal administration. These dosage forms can be used for the treatment of pathologies caused by the aging process, namely, as anticancer agents, life prolongation agents, nephroprotectors, cardioprotectors, cerebroprotectors, hepatoprotectors, antihypertensive drugs and substances that increase the sensitivity of tissues to insulin.

**Example 1. Synthesis of derivative (III)**

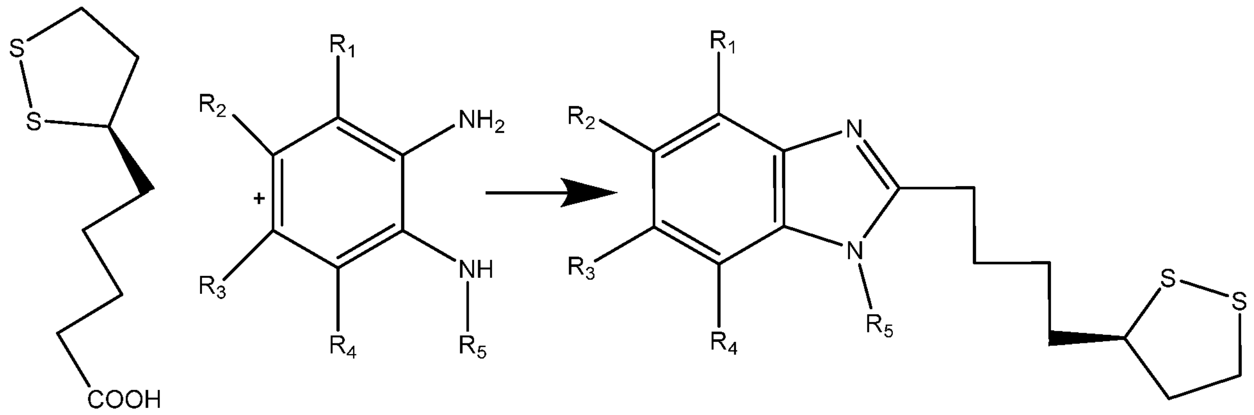

(I) (II) (III)

$R_{1-4}$= H, $-CH_3$, $-C_2H_5$, -Br, - Cl, -I, -F, -OH, -CN, $-NO_2$, -COOH.
$R_5$= H, $-CH_3$, $-C_2H_5$.

A mixture of 2.05 g (0.01 mole) of thioctic acid (I), 1.08 g of 0.01 mole) substituted ortho-phenylenediamine (II), 15 ml of glacial acetic acid and 5 ml of dimethylformamide is boiled for 60 minutes. The solution is cooled, the formed precipitate (III) is filtered off and dried. Crystallized from ethanol. The output is 70-85%.

Instead of unsubstituted (II), its substituted derivatives with $R_{1-4}$= H, $-CH_3$, $-C_2H_5$, -Br, - Cl, -I, - F, -OH, -CN, $-NO_2$, -COOH. $R_5$= H, $-CH_3$, $-C_2H_5$, I.

Instead of glacial acetic acid, a mixture of 1.5 ml of toluene and 5 ml of dimethylformamide can be used. Instead of recrystallization, precipitation from a solution of glacial acetic acid with isopropyl alcohol can be used by adding 5 ml of isopropyl alcohol to the cooled reaction mixture and settling the solution for a day. The precipitate that formed was filtered off and reprecipitated from glacial acetic acid with isopropanol as described above.

**Table 1. Results of NMR $^{13}C$ analysis of some of the synthesized compounds (III) and synthesis yield**

| **Substance code** | **Substituents** | **NMR $^{13}$C**, PPM | **Quantity from theoretical calculation %** |
|---|---|---|---|
| IIIa | $R_{1-5}$= H | 56,3 (CH); 38,5($CH_2$); 138,9(C); 151,4(C); 40,2 ($CH_2$); 115,2 (CH) ; 123,0(CH) ;34,9($CH_2$); 29,2 ($CH_2$); 25,2($CH_2$); 22,1 ($CH_2$) | 85±10 |
| IIIb | $R_{1-3,5}$=H, $R_4$=-COOH | 56,3 (CH); 38,5($CH_2$); 138,9(C); 151,4(C); 129,5 (C); 138,8(C); 40,2($CH_2$); 125,6 (CH) 120,4 (CH) ; 122,9(CH) 166,4(C);;34,9($CH_2$); 29,2($CH_2$); 25,2 ($CH_2$); 22,1 ($CH_2$) | 77±10 |
| IIIc | $R_{1,3-5,}$=H, $R_2$=$-NO_2$ | 56,3 (CH); 38,5($CH_2$); 151,4(C); 142,5(C); 138,7 (C); 40,2($CH_2$); 144,3 (C); 112,9 (CH) ; 116,1(CH); 118,6(CH); 34,9($CH_2$); 29,2($CH_2$); 25,2($CH_2$); 22,1 ($CH_2$) | 80±10 |
| IIId | $R_{1,3,5,}$=H, $R_{2,4}$=$-CH_3$ | 56,3 (CH); 38,5($CH_2$); 151,4(C); 138,7(C); 135,4 (C); 40,2($CH_2$); 126,1(C); 112,3 (CH); 132,6(C); 124,3(CH); 29,2($CH_2$); 16,5($CH_3$); 21,6($CH_3$); 25,2 ($CH_2$); 22,1 ($CH_2$) | 79±10 |
| IIIe | $R_{1,3,5,}$=H, $R_{2,4}$=-Br | 56,3 (CH); 38,5($CH_2$); 148,2(C); 128,8(C); 119,4 (CH); 40,2($CH_2$); 122,3(C); 127,9 (CH); 34,9($CH_2$); 28,8($CH_2$); 136,9(CH); 116,1($CH_2$); 25,2 ($CH_2$); 22,1 ($CH_2$) | 80±10 |

(continued)

| Substance code | Substituents | NMR $^{13}C$, PPM | Quantity from theoretical calculation % |
|---|---|---|---|
| IIIe | $R_{1,3,5,}$=H, $R_{2,4}$=-Cl | 56,3 (CH); 38,5($CH_2$); 151,4(C); 141,7(C); 136,4 (CH); 40,2($CH_2$); 122,1(C); 130,6 (C); 113,9(CH); 123,7(CH); 34,9($CH_2$); 29,2($CH_2$); 25,2 ($CH_2$); 22,1 ($CH_2$) | 85±10 |
| IIIf | $R_{1,3,}$=H, $R_{2,4,5}$=-$CH_3$ | 56,3 (CH); 38,5($CH_2$); 151,4(C); 153,0(C); 138,7 (C); 135,6(C); 40,2($CH_2$); 126,1(C); 112,3(CH); 132,6(C); 124,3(CH); 32,3($CH_3$); 34,9 ($CH_2$); 26,7 ($CH_2$); 16,8($CH_3$); 21,6($CH_3$); 25,2 ($CH_2$); 22,4 ($CH_2$) | 75±10 |
| IIIg | $R_{1,2,4}$=H, $R_3$=-F, $R_5$=-$C_2H_5$ | 56,3 (CH); 38,5($CH_2$); 154,7(C); 137,8(C); 135,8 (C); 40,2($CH_2$); 156,5(C); 102,4(CH); 116,8(CH); 109,9(CH); 40,4($CH_2$); 34,9 ($CH_2$); 27,0 ($CH_2$); 25,2($CH_2$); 22,4($CH_2$); 15,1 ($CH_3$) | 80±10 |
| IIIh | $R_{1,4}$=H $R_3$=-I, $R_2$=-OH; $R_5$=-I | 56,3 (CH); 38,5($CH_2$); 141,5(C); 132,1(C); 139,2 (C); 40,2($CH_2$); 78,9(C); 160,6(C); 125,7(CH); 104,0(CH); 34,9($CH_2$); 27,7 ($CH_2$); 25,2 ($CH_2$); 21,2($CH_2$) | 85±10 |

**Example 2. Synthesis of derivative (V)**

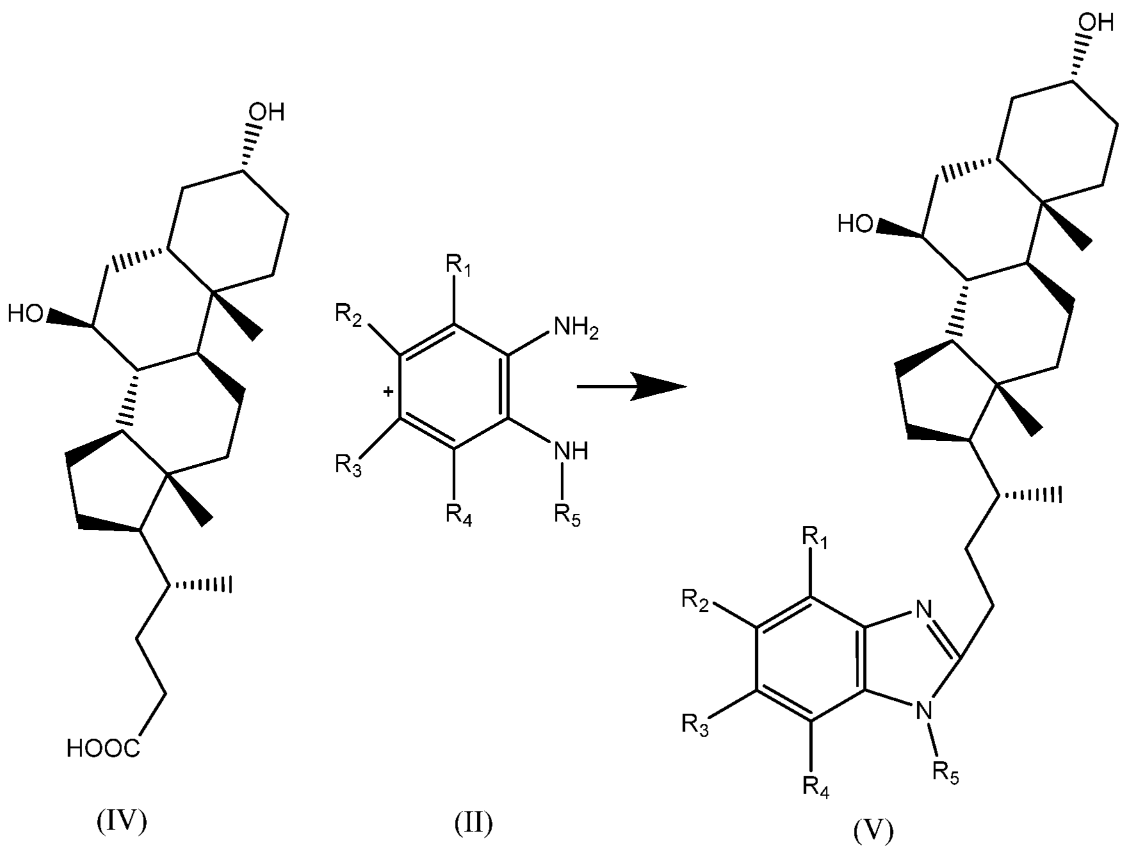

(IV) (II) (V)

$R_{1-4}$= H, -$CH_3$, -$C_2H_5$, -Br, - Cl, -I, -F, -OH, -CN, -$NO_2$, -COOH.

$R_5$= H, $-CH_3$, $-C_2H_5$.

A mixture of 3.9 g (0.01 mole) ursodeoxycholic acid (IV), 1.08 g (0.01 mole) ortho-phenylenediamine (II), 10 ml of glacial acetic acid and 3 ml of dimethylformamide is boiled for 90 minutes. The solution is cooled, the formed precipitate (V) is filtered off and dried. Crystallized from methanol. The output is 70-85%.

Instead of unsubstituted (II), its substituted derivatives with $R_{1-4}$= H, $-CH_3$, $-C_2H_5$, -Br, - Cl, -I, -F, -OH, -CN, $-NO_2$, -COOH. $R_5$= H, $-CH_3$, $-C_2H_5$, I.

Instead of glacial acetic acid, a mixture of 9 ml of toluene and 9 ml of dimethylformamide can be used. Instead of recrystallization, precipitation from a solution of glacial acetic acid with isopropyl alcohol can be used by adding 5 ml of water to the cooled reaction mixture and settling the solution for a day. The precipitate that formed was filtered off and reprecipitated from glacial acetic acid with water as described above.

Table 2. Results of $^{13}C$ NMR analysis of some of the synthesized compounds (V) and synthesis yield

| **Substance code** | **Substituents** | NMR $^{13}C$, PPM | **Quantity from theoretical calculation %** |
|---|---|---|---|
| Va | $R_{1-5}$= H | 151,4 (C); 139,9 (C);138,9 (C); 43,0 (C); 50,4 (CH); 56,3 (CH); 24,5 ($CH_2$); 28,3 ($CH_2$); 68,4 (CH); 71,4 (CH); 115,2 (CH); 40,0 (CH); 40,2 ($CH_2$); 123,0 (CH); 35,8(C); 41,9 (CH); 41,1 (CH); 37,4 (CH); 30,9 ($CH_2$); 21,0 ($CH_2$); 35,6 ($CH_2$); 35,4 (CH); 27,0 ($CH_2$); 13,6 ($CH_3$); 38,9 ($CH_2$); 19,3 ($CH_3$) | 60±10 |
| Vb | $R_{1-3,5}$=H, $R_4$=-COOH | 151,4 (C); 138,8 (C);129,5 (C); 43,0 (C); 50,4 (CH); 56,3 (CH); 24,5 ($CH_2$); 28,3 ($CH_2$); 68,4 (CH); 71,4 (CH); 117,5 (CH); 120,4 (CH); 40,0 (CH); 40,2 ($CH_2$); 125,6 (CH); 122,9 (CH); 35,8(C); 41,9 (CH); 41,1 (CH); 37,4 (CH); 30,9 ($CH_2$); 21,0 ($CH_2$); 35,6 ($CH_2$); 166,4 (C); 35,40 (CH); 27,0 ($CH_2$); 13,6 ($CH_3$); 38,9 ($CH_2$); 19,4 ($CH_3$) | 52±10 |
| Vc | $R_{1,3-5,}$=H, $R_2$=-$NO_2$ | 151,4 (C); 139,8 (C);135,4 (C); 43,0 (C); 50,4 (CH); 56,3 (CH); 24,5 ($CH_2$); 28,3 ($CH_2$); 68,4 (CH); 71,4 (CH); 126,1 (C); 112,3 (CH); 40,0 (CH); 40,2 ($CH_2$); 118,6 (CH); 123,9 (CH); 35,8(C); 41,9 (CH); 41,1 (CH); 37,4 (CH); 30,9 ($CH_2$); 21,0 ($CH_2$); 35,6 ($CH_2$); 35,40 (CH); 27,0 ($CH_2$); 13,6 ($CH_3$); 38,9 ($CH_2$); 19,4 ($CH_3$) | 55±10 |
| Vd | $R_{1,3,5,}$=H, $R_{2,4}$=-$CH_3$ | 151,4 (C); 138,7 (C);133,7 (C); 43,0 (C); 50,4 (CH); 56,3 (CH); 24,5 ($CH_2$); 28,3 ($CH_2$); 68,4 (CH); 71,4 (CH); 137,0 (C); 121,3 (CH); 40,0 (CH); 40,2 ($CH_2$); 132,6 (C); 124,3 (CH); 35,8(C); 41,9 (CH); 41,1 (CH); 37,4 (CH); 30,9 ($CH_2$); 21,0 ($CH_2$); 35,6 ($CH_2$); 35,40 (CH); 27,0 ($CH_2$); 13,6 ($CH_3$); 16,5 ($CH_3$); 21,6 ($CH_3$); 13,5 ($CH_3$) ; 38,9 ($CH_2$); 19,4 ($CH_3$) | 50±10 |
| Ve | $R_{1,3,5,}$=H, $R_{2,4}$=-Cl | 151,4 (C); 141,7 (C);136,4 (C); 43,0 (C); 50,4 (CH); 56,3 (CH); 24,5 ($CH_2$); 28,3 ($CH_2$); 122,1 (C); 130,6 (C); 68,4 (CH); 71,4 (CH); 113,9 (CH); 40,0 (CH); 40,2 ($CH_2$); 123,7 (CH); 35,8(C); 41,9 (CH); 41,1 (CH); 37,4 (CH); 30,9 ($CH_2$); 21,0 ($CH_2$); 35,6 ($CH_2$); 35,40 (CH); 27,0 ($CH_2$); 13,6 ($CH_3$); 13,5 ($CH_3$); 38,9 ($CH_2$); 19,4 ($CH_3$) | 45±10 |
| Ve | $R_{1,3,5,}$=H, $R_{2,4}$=-Br | 151,4 (C); 143,3 (C);140,2 (C); 43,0 (C); 50,4 (CH); 56,3 (CH); 24,5 ($CH_2$); 28,3 ($CH_2$); 112,2 (C); 119,7 (C); 68,4 (CH); 71,4 (CH); 117,7 (CH); 40,0 (CH); 40,2 ($CH_2$); 129,5 (CH); 35,8(C); 41,9 (CH); 41,1 (CH); 37,4 ($CH_2$); 30,9 ($CH_2$); 21,0 | 60±10 |
| | | ($CH_2$); 35,6 ($CH_2$); 35,40 (CH); 27,0 ($CH_2$); 13,6 ($CH_3$); 38,9 ($CH_2$); 19,4 ($CH_3$) | |

(continued)

| Substance code | Substituents | NMR $^{13}C$, PPM | Quantity from theoretical calculation % |
|---|---|---|---|
| Vf | $R_{1,3,}$=H, $R_{2,4,5}$=-$CH_3$ | 153,0 (C); 138,7 (C);135,6 (C); 43,0 (C); 50,4 (CH); 56,3 (CH); 24,5 ($CH_2$); 28,3 ($CH_2$); 68,4 (CH); 71,4 (CH); 126,1 (C); 112,3 (CH); 40,0 (CH); 40,2 ($CH_2$); 132,6 (C); 124,3 (CH); 35,8 (C); 41,9 (CH); 41,1(CH); 37,4 ($CH_2$); 30,9 ($CH_2$); 21,0 ($CH_2$); 35,6 ($CH_2$); 32,3 ($CH_3$); 35,4 (CH); 24,5 ($CH_2$); 13,6 ($CH_3$); 16,8 ($CH_3$); 21,6 ($CH_3$); 13,5 ($CH_3$); 39,2 ($CH_2$); 19,4 ($CH_3$); | 65±10 |
| Vg | $R_{1,2,4}$=H, $R_3$=-F, $R_5$=-$C_2H_5$ | 154,7 (C); 137,8 (C);135,8 (C); 43,0 (C); 50,4 (CH); 56,3 (CH); 24,5 ($CH_2$); 28,3 ($CH_2$); 156,5 (C); 68,4 (CH); 71,4 (CH); 102,3 (CH); 116,8 (CH); 40,0 (CH); 40,2 ($CH_2$); 109,9 (CH); 35,8 (C); 41,9 (CH); 41,1(CH); 37,4 ($CH_2$); 30,9 ($CH_2$); 21,0 ($CH_2$); 35,6 ($CH_2$); 40,4 ($CH_2$); 35,4 (CH); 24,8 ($CH_2$); 13,6 ($CH_3$); 39,2 ($CH_2$); 15,1 ($CH_3$); 19,4 ($CH_3$) | 50±10 |
| Vh | $R_{1,4}$=H $R_3$=-I, $R_2$=-OH; $R_5$=-I | 141,5 (C); 132,0 (C); 139,2 (C); 43,0 (C); 50,4 (CH); 56,3 (CH); 24,5 ($CH_2$); 28,3 ($CH_2$); 78,9 (C); 160,6 (C); 68,4 (CH); 71,4 (CH); 125,7 (CH); 104,0 (CH); 40,0 (CH); 40,2 ($CH_2$); 35,8 (C); 41,9 (CH); 41,9(CH); 41,1 (CH); 37,4 ($CH_2$); 30,9 ($CH_2$); 21,0 ($CH_2$); 35,6 ($CH_2$); 35,4 (CH); 25,5 ($CH_2$); 13,5 ($CH_3$); 38,0 ($CH_2$); 19,4 ($CH_3$) | 55±10 |

**Example 3. Synthesis of derivative (VII)**

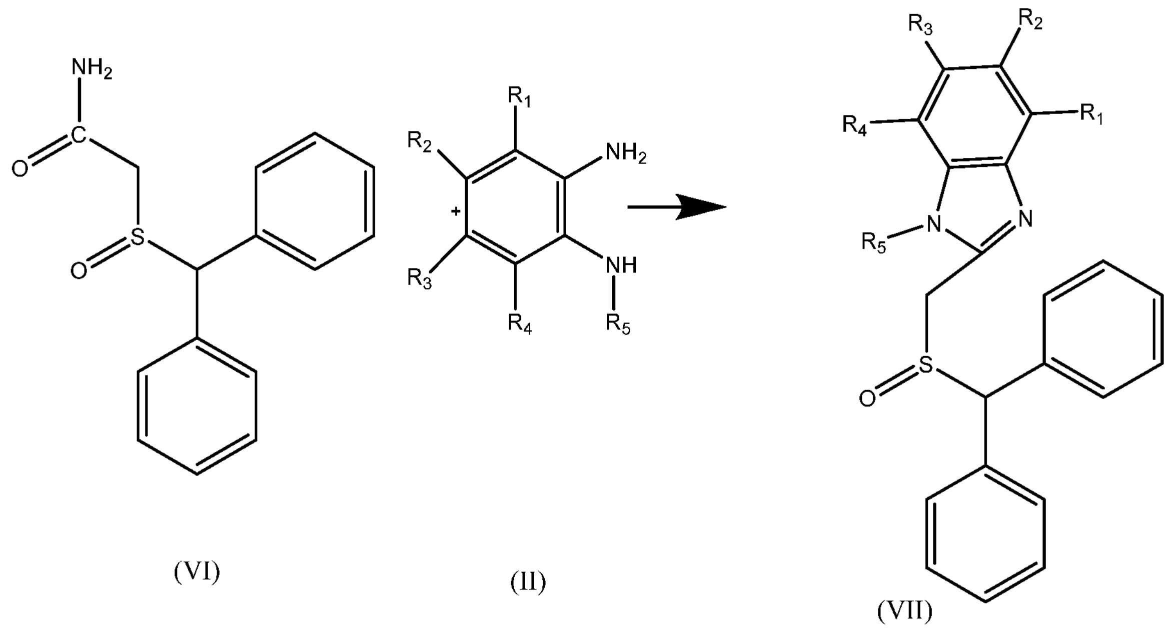

(VI) (II) (VII)

$R_{1-4}$= H, -$CH_3$ -$C_2H_5$, -Br, - Cl, -I, -F, -OH, -CN, -$NO_2$, -COOH.
$R_5$= H, -$CH_3$, -$C_2H_5$.

A mixture of 2.7 g (0.01 mole) of modafinil (VI), 1.08 g (0.01 mole) of ortho-phenylenediamine (II), 5 ml of glacial acetic acid and 3 ml of dimethylformamide is boiled for 90 minutes. The solution is cooled, the precipitate (VII) formed is filtered off and dried. Crystallized from methanol. The output is 60-65%.

Instead of unsubstituted (II), its substituted derivatives with $R_{1-4}$= H, $-CH_3$, $-C_2H_5$, -Br, - Cl, -I, - F, -OH, -CN, $-NO_2$, -COOH. $R_5$= H, $-CH_3$, $-C_2H_5$, I.

Instead of glacial acetic acid, a mixture of 5 ml of toluene and 5 ml of dimethylformamide can be used. Instead of recrystallization, precipitation from a solution of glacial acetic acid can be used by adding 5 ml of water to the cooled reaction mixture and settling the solution for a day. The precipitate that formed was filtered off and reprecipitated from glacial acetic acid with water as described above. Table 3 shows the analysis data for some of the synthesized compounds.

Table 3. Results of NMR $^{13}C$ analysis of some of the synthesized compounds (VII) and synthesis yield

| **Substance code** | **Substituents** | NMR $^{13}C$, PPM | **Quantity from theoretical calculation %** |
|---|---|---|---|
| VIIa | $R_{1-5}$= H | 141,5 (C); 138,9 (C); 115,2 (CH); 135,2 (CH); 123,1 (CH); 130,0 (CH); 129,2 (CH); 126,2 (CH); 49,8 ($CH_2$); 67,2 (CH) | 63±10 |
| VIIb | $R_{1-3,5}$=H, $R_4$=-COOH | 141,5 (C); 138,8 (C); 129,5 (C); 117,5 (C); 120,4 (CH); 135,2 (C); 125,6 (CH); 122,9 (CH); 130,1 (CH); 129,2 (CH); 126,2 (CH); 49,8 ($CH_2$) | 67±10 |
| VIIc | $R_{1,3-5,}$=H, $R_2$=-$NO_2$ | 141,5 (C); 145,0 (C); 144,3 (C); 112,9 (CH); 116,1 (CH); 135,2 (C); 118,6 (CH); 130,1(CH); 129,2 (CH); 126,2 (CH); 49,8 ($CH_2$) | 55±10 |
| VIId | $R_{1,3,5,}$=H, $R_{2,4}$=-$CH_3$ | 141,5 (C); 138,7 (C); 135,4 (C); 126,1 (C); 112,3 (CH); 132,6 (C); 135,2 (C); 124,3 (CH); 130,1 (CH); 129,2 (CH); 126,2 (CH); 49,8 ($CH_2$) | 50±10 |
| VIIe | $R_{1,3,5,}$=H, $R_{2,4}$=-Cl | 141,5 (C); 141,7 (C); 136,4 (C); 122,1 (C); 130,6 (C); 113,9 (CH); 135,2 (C); 123,7 (CH); 130,1 (CH); 129,2 (CH); 126,2 (CH); 49,8 ($CH_2$); 67,2 (CH); | 66±10 |
| VIIf | $R_{1,3,5,}$=H, $R_{2,4}$=-Br | 141,5 (C); 143,3 (C); 140,2 (C); 112,1 (C); 119,7 (C); 117,7 (CH); 135,2 (C); 129,5 (CH); 130,1 (CH); 129,2 (CH); 126,2 (CH); 49,8 ($CH_2$); 67,2 (CH); | 60±10 |
| VIIg | $R_{1,3,}$=H, $R_{2,4,5}$=-$CH_3$ | 141,5 (C); 138,7 (C); 135,6 (C); 126,1 (C); 112,3 (CH); 132,6 (C); 135,2 (C); 124,3 (CH); 130,1 (CH); 129,2 (CH); 126,2 (CH); 47,3 ($CH_2$); 67,2 (CH); 32,5 ($CH_3$); 16,8 ($CH_3$); 21,6 ($CH_3$); | 72±10 |
| VIIh | $R_{1,2,4}$=H, $R_3$=-F, $R_5$=-$C_2H_5$ | 141,5 (C); 137,8 (C); 135,8 (C); 156,5 (C); 102,4 (CH); 116,8 (CH); 135,2 (C); 109,9 | 58±10 |
| | | (CH); 130,1(CH); 129,2 (CH); 126,2 (CH); 47,6 ($CH_2$); 67,2 (CH); 40,6 ($CH_2$); 15,1 ($CH_3$) | |
| VIIi | $R_{1,4}$=H $R_3$=-I, $R_2$=-OH; $R_5$=-I | 141,5 (C); 132,1 (C); 139,2 (C); 78,9 (C); 160,6 (C); 125,7 (CH); 104,0 (CH); 135,2 (C); 109,9 (CH); 130,1(CH); 129,2 (CH); 126,2 (CH); 48,3 ($CH_2$); 67,2 (CH) | 55±10 |

**Example 4. Synthesis of derivative (IX)**

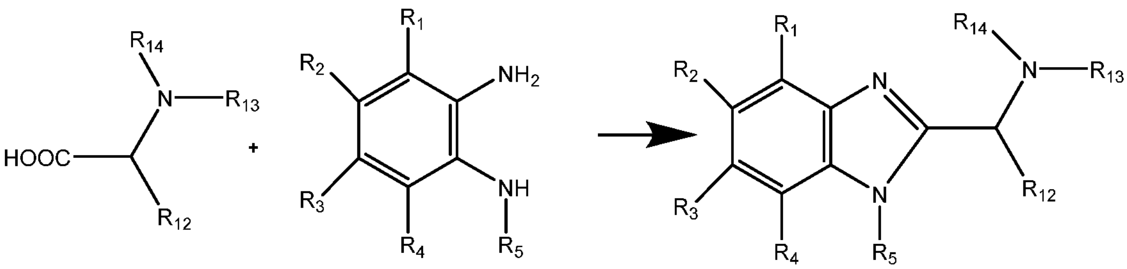

(VIII) (II) (IX)

$R_{1-4}$= H, $-CH_3$, $-C_2H_5$, -Br, - Cl, -1, -F, -OH, -CN, $-NO_2$, -COOH;
$R_5$= H, $-CH_3$, $-C_2H_5$;
$R_{12}$=$-CH_3$;
$R_{13,14}$= H, $-CH_3$, $-C_2H_5$;

A mixture of 0.01 mole of amino acid (VIII), 0.01 mole of ortho-phenylenediamine (II), 10 ml of glacial acetic acid is boiled for 60 minutes. The solution is cooled, 7 ml of isopropyl alcohol is added, a day later the precipitate (IX) is filtered off and dried. Re-precipitate from glacial acetic acid with isopropanol as described above. The output is 70-85%.
Instead of unsubstituted (II), its substituted derivatives with c $R_{1-4}$= H, $-CH_3$, $-C_2H_5$, - Br, - Cl, -I, -F, -OH, -CN, $-NO_2$, -COOH. $R_5$= H, $-CH_3$, $-C_2H_5$, I. As amino acid (VIII), unsubstituted or substituted derivatives of glutamic, aspartic acids, arginine, lysine, amides of glutamic and aspartic acids, valine, tryptophan, alanine can be used.
Instead of glacial acetic acid, a mixture of 5 ml of toluene and 5 ml of dimethylformamide can be used. Instead of recrystallization, precipitation from a solution of glacial acetic acid can be used by adding 5 ml of water to the cooled reaction mixture and settling the solution for a day. The precipitate that formed was filtered off and reprecipitated from glacial acetic acid with water as described above. Table 4 shows the analysis data for some of the synthesized compounds.

**Table 4. Results of NMR $^{13}C$ analysis of some of the synthesized compounds (IX) and synthesis yield**

| **Substance code** | **Substituents** | NMR $^{13}C$, PPM | **Quantity from theoretical calculation %** |
|---|---|---|---|
| IXa | $R_{1-5,13,14}$= H; $R_{12}$= 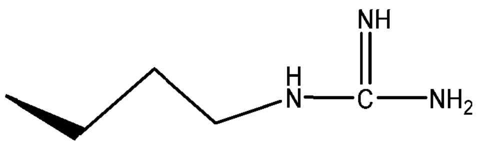 | 141,5 (C); 138,9 (C); 115,2 (CH); 123,0 (CH); 158,0 (CH); 55,1 (CH); 41,9 (CH); 36,5 (CH); 24,8 ($CH_2$) | 65±10 |
| IXb | $R_{1-5,13,14}$= H; $R_{12}$= 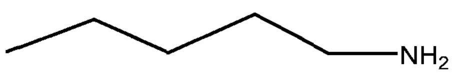 | 141,5 (C); 138,9 (C); 115,2 (CH); 123,0 (CH); 158,0 (CH); 55,1 (CH); 42,0 (CH); 36,5 (CH); 27,2 ($CH_2$) | 60±10 |
| IXc | $R_{1-5,13,14}$= H; $R_{12}$= 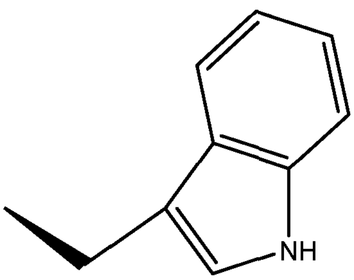 | 141,5 (C); 138,9 (C); 136,5 (CH); 123,0 (CH); 127,4 (C); 110,8 (C); 115,2 (CH); 111,2 (CH); 118,8 (CH); 123,0 (CH); 121,7 (CH); 119,8 (CH); 58,6 (CH); 37,8 ($CH_2$) | 75±10 |

(continued)

| **Substance code** | **Substituents** | NMR $^{13}C$, PPM | **Quantity from theoretical calculation %** |
|---|---|---|---|
| IXd | $R_{1-5,13,14}$= H; $R_{12}$= 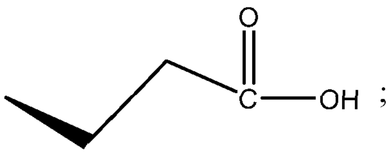 ; | 141,5 (C); 138,9 (C); 115,2 (CH); 123,0 (CH); 178,4 (C); 54,8 (CH); 30,6 (CH); 33,0 ($CH_2$) | 79±10 |
| IXe | $R_{1-5,13,14}$= H; $R_{12}$= 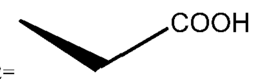 | 141,5 (C); 138,9 (C); 115,2 (CH); 123,0 (CH); 172,1 (C); 50,2 (CH); 44,8 ($CH_2$) | 66±10 |
| IXf | $R_{1-5,13,14}$= H; $R_{12}$= 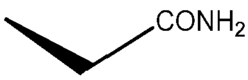 | 141,5 (C); 138,9 (C); 115,2 (CH); 123,0 (CH); 174,3 (C); 50,8 (CH); 46,4 ($CH_2$) | 80±10 |
| IXg | $R_{1-5,13,14}$= H; $R_{12}$= 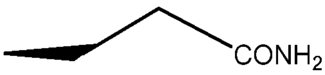 | 141,5 (C); 138,9 (C); 115,2 (CH); 123,0 (CH); 74,3 ($CH_2$); 55,4 (CH); 21,6 ($CH_2$); 35,4 ($CH_2$) | 75±10 |

**Example 5. Synthesis of derivative (XI)**

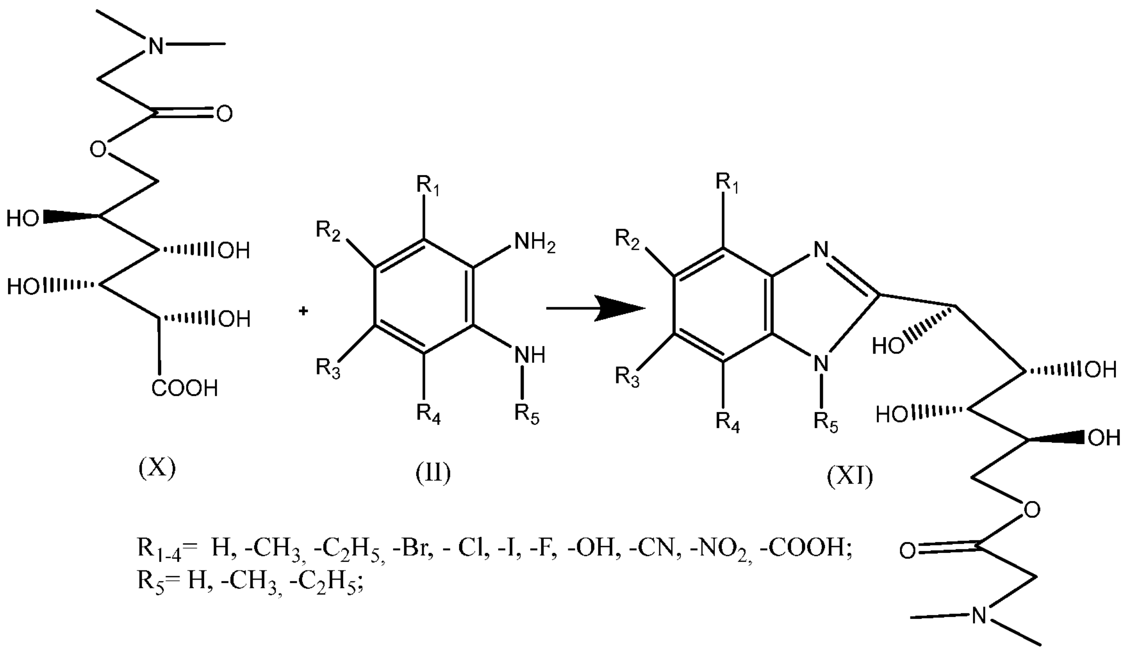

(X) (II) (XI)

$R_{1-4}$= H, $-CH_3$, $-C_2H_5$, -Br, - Cl, -I, -F, -OH, -CN, $-NO_2$, -COOH;
$R_5$= H, $-CH_3$, $-C_2H_5$;

A mixture of 0.01 mole of pangamic acid (X), 0.01 mole of ortho-phenylenediamine (II), 10 ml of glacial acetic acid is boiled for 30 minutes. The solution is cooled, 5 ml of isopropyl alcohol is added, a day later the precipitate (XI) is filtered off and dried. Re-precipitate from glacial acetic acid with isopropanol as described above. The yield is 60-75%.

Instead of unsubstituted (II), its substituted derivatives with $R_{1-4}$= H, $-CH_3$, $-C_2H_5$, -Br, - Cl, -I, -F, -OH, -CN, $-NO_2$, -COOH. $R_5$= H, $-CH_3$, $-C_2H_5$, I. Instead of pangamic acid (X), biotin, pantothenic acid, folic acid, cyanocobalamin

can be used.

Instead of glacial acetic acid, a mixture of 5 ml of toluene and 5 ml of dimethylformamide can be used. To purify the product, precipitation from a solution of glacial acetic acid can be used by adding 5 ml of water to the cooled reaction mixture and settling the solution for a day. The precipitate that formed was filtered off and reprecipitated from glacial acetic acid with water as described above. Table 5 shows the analysis data for some of the synthesized compounds.

**Table 5. Results of NMR $^{13}C$ analysis of some of the synthesized compounds (XI) and synthesis yield**

| **Substance code** | **Substituents** | NMR $^{13}C$, PPM | **Quantity from theoretical calculation %** |
|---|---|---|---|
| XIa | $R_{1-5}$= H | 141,5 (C); 138,9 (C); 115,2 (CH); 123,1 (CH); 171,2 (C); 66,9 (CH); 70,3 (CH); 69,1 (CH); 69,3 (CH); 65,0 ($CH_2$); 47,2 ($CH_2$); 53,9 ($CH_3$) | 63±10 |
| XIb | $R_{1-3,5}$=H, $R_4$=-COOH | 141,5 (C); 138,8 (C); 117,5 (C); 120,4 (CH); 125,6 (CH); 122,9 (CH); 166,4 (C); 171,2 (C); 66,9 (CH); 70,3 (CH); 69,1 (CH); 69,3 (CH); 65,0 ($CH_2$); 47,2 ($CH_2$); 53,9 ($CH_3$) | 60±10 |
| XIc | $R_{1,3-5,}$=H, $R_2$=-$NO_2$ | 141,5 (C); 139,8 (C); 133,7 (C); 137,0 (C); 121,3 (CH); 118,6(CH); 123,9 (CH); 171,2 (C); 66,9 (CH); 70,3 (CH); 69,1 (CH); 69,3 (CH); 65,0 ($CH_2$); 47,2 ($CH_2$); 53,9 ($CH_3$) | 75±10 |
| XId | $R_{1,3,5,}$=H, $R_{2,4}$=-$CH_3$ | 141,5 (C); 138,7 (C); 135,4 (C); 126,1 (C); 112,3 (CH); 132,6(C); 124,3 (CH); 171,2 (C); 66,9 (CH); 70,3 (CH); 69,1 (CH); 69,3 (CH); 65,0 ($CH_2$); 47,2 ($CH_2$); 53,9 ($CH_3$) ; 16,5 ($CH_3$); 21,6 ($CH_3$) | 50±10 |
| XIe | $R_{1,3,5,}$=H, $R_{2,4}$=-Cl | 141,5 (C); 141,7 (C); 136,4 (C); 122,1 (C); 130,6 (C); 113,9 (CH); 135,2 (C); 123,7 (CH); 130,1(CH); 129,2 (CH); 126,2 (CH); 49,8 ($CH_2$); 67,2 (CH); | 66±10 |
| XIf | $R_{1,3,5,}$=H, $R_{2,4}$=-Br | 141,5 (C); 143,3 (C); 140,2 (C); 112,1 (C); 119,7 (C); 117,7 (CH); 135,2 (C); 129,5 (CH); 130,1(CH); 129,2 (CH); 126,2 (CH); 49,8 ($CH_2$); 67,2 (CH); | 60±10 |
| XIg | $R_{1,3,}$=H, $R_{2,4,5}$=-$CH_3$ | 141,5 (C); 138,7 (C); 135,6 (C); 126,1 (C); 112,3 (CH); 132,6 (C); 135,2 (C); 124,3 (CH); 130,1(CH); 129,2 (CH); 126,2 (CH); 47,3 ($CH_2$); 67,2 (CH); 32,5 ($CH_3$); 16,8 ($CH_3$); 21,6 ($CH_3$); | 72±10 |
| XIh | $R_{1,2,4}$=H, $R_3$=-F, $R_5$=-$C_2H_5$ | 141,5 (C); 137,8 (C); 135,8 (C); 156,5 (C); 102,4 (CH); 116,8 (CH); 135,2 (C); 109,9 (CH); 130,1 (CH); 129,2 (CH); 126,2 (CH); 47,6 ($CH_2$); 67,2 (CH); 40,6 ($CH_2$); 15,1 ($CH_3$) | 58±10 |
| XIi | $R_{1,4}$=H $R_3$=-I, $R_2$=-OH; $R_5$=-I | 141,5 (C); 132,1 (C); 139,2 (C); 78,9 (C); 160,6 (C); 125,7 (CH); 104,0 (CH); 135,2 (C); 109,9 (CH); 130,1(CH); 129,2 (CH); 126,2 (CH); 48,3 ($CH_2$); 67,2 (CH) | 55±10 |

**Example 6. Synthesis of derivative (XIII)**

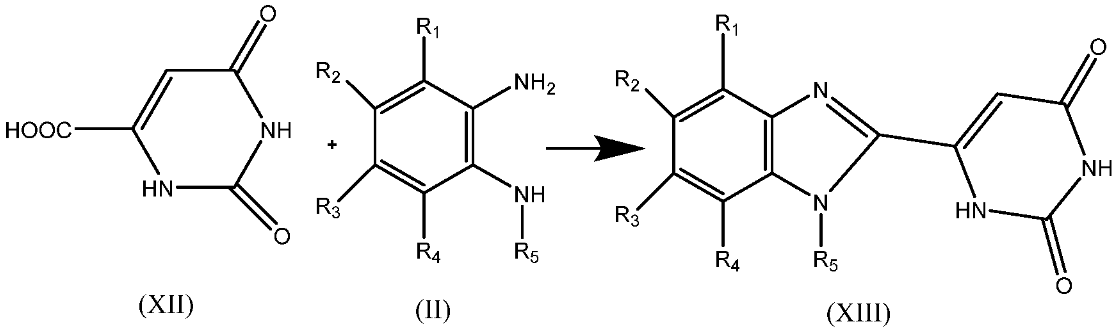

(XII) (II) (XIII)

$R_{1-4}$= H, $-CH_3$, $-C_2H_5$, -Br, - Cl, -I, -F, -OH, -CN, $-NO_2$, -COOH;
$R_5$= H, $-CH_3$, $-C_2H_5$;

A mixture of 0.01 mole of orotic acid (XII), 0.01 mole of ortho-phenylenediamine (II), 10 ml of glacial acetic acid is boiled for 90 minutes. The solution is cooled, 5 ml of isopropyl alcohol is added, after a day the precipitate (XIII) is filtered off and dried. Re-precipitate from glacial acetic acid with isopropanol as described above. The yield is 65-75%.

Instead of unsubstituted (II), its substituted derivatives with $R_{1-4}$= H, $-CH_3$, $-C_2H_5$, -Br, - Cl, -I, - F, -OH, -CN, $-NO_2$, -COOH. $R_5$= H, $-CH_3$, $-C_2H_5$, I.

Instead of glacial acetic acid, a mixture of 5 ml of toluene and 5 ml of dimethylformamide can be used. To purify the product, precipitation from a solution of glacial acetic acid can be used by adding 5 ml of water to the cooled reaction mixture and settling the solution for a day. The precipitate that formed was filtered off and reprecipitated from glacial acetic acid with water as described above. Table 6 shows the analysis data of some of the synthesized compounds.

**Table 6. Results of NMR $^{13}$C analysis of some of the synthesized compounds (XIII) and synthesis yield**

| **Substance code** | **Substituents** | NMR $^{13}$C, PPM | **Quantity from theoretical calculation %** |
|---|---|---|---|
| XIIIa | $R_{1-5}$= H | 141,5 (C); 138,9 (C); 150,7 (CH); 163,5 (CH); 154,7 (CH); 115,2 (CH); 95,3 (CH); 123,0 (CH) | 65±10 |
| XIIIb | $R_{1-3,5}$=H, $R_4$=-COOH | 141,5 (C); 138,8 (C); 129,5 (C); 150,7 (C); 163,5 (C); 154,7 (C); 117,5 (C); 120,4 (CH); 95,3 (CH); 125,6 (CH); 122,9 (CH); 166,4 (C) | 75±10 |
| XIIIc | $R_{1,3-5,}$=H, $R_2$=$-NO_2$ | 141,5 (C); 139,8 (C); 133,7 (C); 150,7 (C); 163,5 (C); 154,7 (C); 137,0 (C); 121,3 (CH); 95,3 (CH); 118,6 (CH); 123,9 (CH) | 75±10 |
| XIIId | $R_{1,3,5,}$=H, $R_{2,4}$=$-CH_3$ | 141,5 (C); 138,7 (C); 135,4 (C); 150,7 (C); 163,5 (C); 154,7 (C); 126,1 (C); 112,3 (CH); 132,6 (C); 95,3 (CH); 124,3 (CH); 16,5 ($CH_3$); 21,6 ($CH_3$) | 80±10 |
| XIIIe | $R_{1,3,5,}$=H, $R_{2,4}$=-Cl | 141,5 (C); 141,7 (C); 136,4 (C); 122,1 (C); 130,6 (C); 150,7 (C); 163,5 (C); 154,7 (CH); 113,9 (CH); 123,7 (CH); 95,3 (CH) | 78±10 |
| XIIIf | $R_{1,3,5,}$=H, $R_{2,4}$=-Br | 141,5 (C); 143,3 (C); 140,2 (C); 112,1 (C); 119,7 (C); 150,7 (CH); 163,5 (C); 154,7 (CH); 117,7 (CH); 129,2 (CH); 95,3 (CH) | 60±10 |
| XIIIg | $R_{1,3,}$=H, $R_{2,4,5}$=$-CH_3$ | 141,5 (C); 138,7 (C); 135,6 (C); 150,7 (C); 163,5 (C); 154,7 (C); 126,1 (C); 112,3 (CH); 132,6 (CH); 95,3 (CH); 124,3 (CH); 34,4 ($CH_3$); 16,8 ($CH_3$); 21,6 ($CH_3$); | 80±10 |

(continued)

| Substance code | Substituents | NMR $^{13}C$, PPM | Quantity from theoretical calculation % |
|---|---|---|---|
| XIIIh | $R_{1,2,4}$=H, $R_3$=-F, $R_5$=-$C_2H_5$ | 141,5 (C); 134,5 (C); 135,8 (C); 156,5 (C); 150,7 (CH); 163,5 (CH); 154,7 (C); 102,4 (CH); 116,8 (CH); 95,3 (CH); 109,9 (CH); 40,5 ($CH_2$); 15,2 ($CH_3$) | 58±10 |
| XIIIi | $R_{1,4}$=H $R_3$=-I, $R_2$=-OH; $R_5$=-I | 141,5 (C); 132,1 (C); 139,2 (C); 78,9 (C); 150,7 (C); 163,5 (CH); 160,6 (CH); 154,7 (C); 125,7 (CH); 104,0(CH); 95,3 (CH) | 55±10 |

**Example 7. Synthesis of derivative (XIV)**

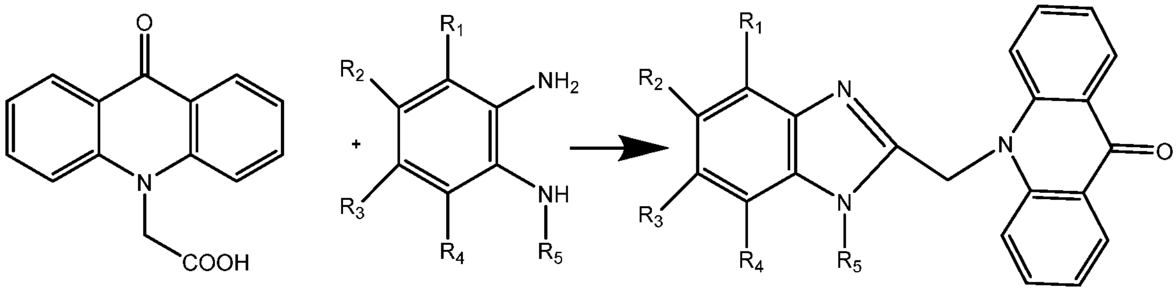

(XIV) (II) (XV)

$R_{1-4}$= H, $-CH_3$, $-C_2H_5$, -Br, - Cl, -1, -F, -OH, -CN, $-NO_2$, -COOH;
$R_5$= H, $-CH_3$, $-C_2H_5$;

A mixture of 0.01 mole of acridoneacetic acid (XIV), 0.01 mole of ortho-phenylenediamine (II), 10 ml of glacial acetic acid is boiled for 90 minutes. The solution is cooled, 5 ml of isopropyl alcohol is added, after a day the precipitate (XV) is filtered off and dried. Re-precipitate from glacial acetic acid with isopropanol as described above. The yield is 55-60%.

Instead of unsubstituted (II), its substituted derivatives with $R_{1-4}$= H, $-CH_3$, $-C_2H_5$, -Br, - Cl, -I, -F, -OH, -CN, $-NO_2$, -COOH. $R_5$= H, $-CH_3$, $-C_2H_5$, I. Instead of glacial acetic acid, a mixture of 5 ml of toluene and 5 ml of dimethylformamide can be used. To purify the product, precipitation from a solution of glacial acetic acid can be used by adding 5 ml of water to the cooled reaction mixture and settling the solution for a day. The precipitate that formed was filtered off and reprecipitated from glacial acetic acid with water as described above. Table 7 shows the analysis data of some of the synthesized compounds.

**Table 7. Results of NMR $^{13}C$ analysis of some of the synthesized compounds (XV) and synthesis yield**

| Substance code | Substituents | NMR $^{13}C$, PPM | Quantity from theoretical calculation % |
|---|---|---|---|
| XVa | $R_{1-5}$= H | 141,5 (C); 138,9 (C); 175,7 (C); 144,4 (C); 115,2 (CH); 121,7 (C); 116,2 (CH); 123,0 (CH); 126,5 (CH); 133,3 (CH); 121,5 (CH); 61,4 ($CH_2$) | 57±3 |
| XVb | $R_{1-3,5}$=H, $R_4$=-COOH | 141,5 (C); 138,9 (C); 129,5 (C); 175,7 (C); 144,4 (C); 117,5 (CH); 120,4 (CH); 121,7 (C); 116,2 (CH); 125,6 (CH); 122,9 (CH); 126,5 (CH); 133,3 (CH); 121,5 (CH); 166,4 (C); 61,4 ($CH_2$) | 52±10 |

(continued)

| Substance code | Substituents | NMR $^{13}C$, PPM | Quantity from theoretical calculation % |
|---|---|---|---|
| XVc | $R_{1,3-5,}$=H, $R_2$=-$NO_2$ | 141,5 (C); 142,5 (C); 145, 0 (C);144,4 (C); 112,9 (CH); 121,7 (C); 116,1 (CH); 121,7 (C); 116,2 (CH); 118,6 (CH); 126,5 (CH); 133,3 (CH); 121,5 (CH); 61,4 ($CH_2$) | 55±5 |
| XVd | $R_{1,3,5}$=H, $R_{2,4}$=-$CH_3$ | 141,5 (C); 141,7 (C); 136,4 (C); 122, 1 (C); 130,6 (C); 175,7 (C); 144,4 (C); 113,9 (CH); 121,7 (C); 123,7 (CH); 116,2 (CH); 126,5 (CH); 133,3 (CH); 121,5 (CH); 61,4 ($CH_2$) | 60±10 |
| XVe | $R_{1,3,5}$=H, $R_{2,4}$=-Cl | 141,5 (C); 141,7 (C); 136,4 (C); 122,1 (C); 130,6 (C); 150,7 (C); 163,5 (C); 154,7 (CH); 113,9 (CH); 123,7 (CH); 95,3 (CH) | 70±10 |
| XVf | $R_{1,3,5,}$=H, $R_{2,4}$=-Br | 141,5 (C); 143,3 (C); 140,2 (C); 112, 2 (C); 119,7 (C); 175,7 (C); 144,4 (C); 117,7 (CH); 121,7 (C); 129,5 (CH); 116,2 (CH); 126,5 (CH); 133,3 (CH); 121,5 (CH); 61,4 ($CH_2$) | 55±10 |
| XVg | $R_{1,3,}$=H, $R_{2,4,5}$=-$CH_3$ | 148,1 (C); 138,7 (C); 135,6 (C); 175, 7 (C); 144,4 (C); 126,1 (C); 112,3 ($CH_3$); 121,7 (C); 132,6 (CH); 116,2 (CH); 124,3 (CH); 126,5 (CH); 133,3 (CH); 121,5 (CH); 32,0 ($CH_3$); 16,8 ($CH_3$); 21,6 ($CH_3$); 58,9 ($CH_2$) | 65±10 |
| XVh | $R_{1,2,4}$=H, $R_3$=-F, $R_5$=-$C_2H_5$ | 148,1 (C); 137,8 (C); 135,8 (C); 156, 54 (C); 175,7 (C); 144,4 (C); 102,4 (CH); 116,8 (CH); 121,7 (C); 116,2 (CH); 109,9 (CH); 126,5 (CH); 116,3 (CH); 126,5 (CH); 133,3 (CH); 121,5 (CH); 40,0 ($CH_2$); 59,2 ($CH_2$); 15,1 ($CH_3$) | 55±10 |
| XVi | $R_{1,4}$=H, $R_3$=-I, $R_2$=-OH; $R_5$=-I | 141,5 (C); 132,1 (C); 139,2 (C); 78, 9 (C); 175,7 (C); 160,6 (C); 144,4 (C); 125,7 (CH); 104,0 (CH); 121,7 (C); 116,2 (CH); 126,5 (CH); 116,2 (CH); 126,5 (CH); 133,3 (CH); 121,5 (CH); 59,9 ($CH_2$) | 50±10 |

**Example 8. Geroprotective action of the components**

The main geroprotective effect of imidazoline receptor activators is an increase in the lifespan of animals. The most studied model is the survival model of Drosophila due to their short lifespan. Drosophila melanogaster lines selected for differences in reproductive function.

Since 1966 the selection of related Drosophila lines was carried out for reproductive function (sexual activity of males). The selection was accompanied by close inbreeding - individual crosses in each generation of full brothers and sisters. In the process of selection, a series of lines differing in breeding characteristics (BA-, HA-, HA +) was repeatedly obtained by means of return selection. This selection led to the acquisition by low-level lines of a complex of genetically controlled changes, the most important of which affected the neuroendocrine system of flies, which became the subject of special studies. In the lines laid down from the natural population of "GL", selection was carried out for embryonic mortality, accompanied by close inbreeding. As a result, contrasting inbred lines were obtained: with high (HEM line) and low embryonic mortality (LEM line).

At the same time, a sample of flies from the natural population of the GL was maintained without selection in mass crops. It was found that the lines of HEM and GL are characterized by threefold differences in the number of viable offspring, while not differing in fertility, determined by the number of laid eggs per unit of time. In the HEM line, 81% of eggs stop developing already at the early stages of ontogenesis, that is, this line is characterized by a high frequency of early dominant lethal (EDL), and it increases from 65% on the first day of laying to 95% on the fourth day. No differences were found between the studied lines in terms of late dominant lethality. The performed genetic analysis showed that the frequency and dynamics of the occurrence of EDL in the HEM line is completely determined by the genotype of the female. In addition, it was found that a system of balanced lethal mutations arose in the HEM line by the 86th generation

of directed selection, creating permanent heterozygosity on a small section of the second chromosome.

The initial number of adults during the first six hours after emergence was subjected to ether anesthesia and placed in individual glass cups (from 5 to 10 virgin females and males in each). The cultures were assigned individual numbers. Subsequently, the dead individuals were regularly counted visually in each glass separately, without ether anesthesia, after which the surviving flies were transferred to a fresh medium, while maintaining the serial number of the glass. The size of each cohort ranged from 100 to 500 individuals. The following parameters were calculated: the median of the survival curve - as MT50 in the equation of the curve (hereinafter MLS - median life span)

$$Y = \frac{100}{1+10^{((MT_{50}-X)\times HS))}}$$

where Y is the percentage of living individuals of the cohort, X is the age of the cohort, MT50 and HS are the parameters of the regression equation. The slope of the survival curve is like HS - Hill Slope in the same equation. It is easy to see that the MT50 parameter is a close analogue of the average life expectancy. Moreover, it is calculated using the least squares method - accordingly, it is the standard regression coefficient, and such coefficients can be compared using the Fisher's F-test.

Parameter HS - statistically estimates the slope of the curve, which means that it indirectly estimates the maximum life span. Since this curve asymptotically tends to zero with increasing age of the cohort, we also propose to estimate the point on the X axis, which corresponds to the value of the function (proportion of living individuals) equal to 0.1%. We will call this age "the expected maximum life duration " (EMLD). The coefficients of determination of the regression model in all cases exceed 90%. The table below shows the MLS - median life span for the tested compounds, which were introduced into insect feed by spraying a 1% solution.

**Table 8. Parameters of survival curves in different variants of the experiment for the synthesized benzimidazoles**

| **Substance** | **Females** | | **Males** | |
|---|---|---|---|---|
| | **MLS (day)** | **EMLD (day)** | **MLS (day)** | **EMLD (day)** |
| **Control** | 26±2 | 30,0 | 21±1 | 23,0 |
| IIIa | 35±5 | | 33±5 | |
| IIIb | 44±5 | | 42±6 | |
| IIIc | 32±6 | | 30±5 | |
| IIId | 37±5 | | 39±6 | |
| IIIe | 38±5 | | 36±5 | |
| IIIf | 32±5 | | 30±5 | |
| IIIg | 30±5 | | 30±5 | |
| **IIIh** | **52±5** | | **57±5** | |
| IIIi | 42±6 | | 42±6 | |
| Va | 40±5 | | 44±6 | |
| Vb | 33±5 | | 37±5 | |
| Vc | 37±5 | | 35±5 | |
| Vd | 43±6 | | 42±6 | |
| Ve | 40±5 | | 38±5 | |
| Vf | 43±5 | | 42±6 | |
| Vg | 44±6 | | 40±6 | |
| Vh | 43±5 | | 44±6 | |
| Vi | 32±5 | | 31±5 | |

(continued)

| Substance | Females | | Males | |
|---|---|---|---|---|
| | MLS (day) | EMLD (day) | MLS (day) | EMLD (day) |
| VIIa | 37±5 | | 34±5 | |
| VIIb | 38±5 | | 33±5 | |
| VIIc | 35±5 | | 38±5 | |
| VIId | 33±5 | | 37±5 | |
| VIIe | 30±5 | | 33±5 | |
| VIIf | 36±5 | | 37±5 | |
| VIIg | 33±5 | | 39±5 | |
| VIIh | 39±5 | | 43±6 | |
| VIIi | 42±6 | | 48±7 | |
| **IXa** | **54±8** | | **59±8** | |
| **IXb** | **55±6** | | **57±8** | |
| IXc | 34±5 | | 42±5 | |
| IXd | 37±5 | | 36±5 | |
| IXe | 35±5 | | 38±5 | |
| IXf | 22±5 | | 26±5 | |
| IXg | 43±6 | | 44±6 | |
| **XIa** | **50±7** | | **57±7** | |
| XIb | 43±6 | | 44±6 | |
| XIc | 40±6 | | 42±6 | |
| XId | 33±5 | | 35±5 | |
| XIe | 36±5 | | 38±5 | |
| XIf | 37±5 | | 36±5 | |
| XIg | 35±5 | | 39±5 | |
| XIh | 40±6 | | 44±6 | |
| XIi | 47±6 | | 48±7 | |
| XIIIa | 40±6 | | 38±5 | |
| XIIIb | 35±5 | | 35±5 | |
| XIIIc | 55±7 | | 59±7 | |
| XIIId | 44±6 | | 45±6 | |
| XIIIe | 40±5 | | 39±5 | |
| XIIIf | 45±6 | | 45±6 | |
| XIIIg | 32±5 | | 33±5 | |
| **XIIIh** | **54±8** | | **59±8** | |
| XIIIi | 43±6 | | 45±6 | |
| **XVa** | **49±7** | | **55±9** | |
| XVb | 47±6 | | 48±7 | |
| XVc | 45±6 | | 43±6 | |

(continued)

| Substance | Females | | Males | |
|---|---|---|---|---|
| | **MLS (day)** | **EMLD (day)** | **MLS (day)** | **EMLD (day)** |
| XVd | 49±8 | | 48±7 | |
| XVe | 44±6 | | 46±5 | |
| XVf | 40±5 | | 42±6 | |
| **XVg** | **67±9** | | **66±9** | |
| **XVh** | **55±8** | | **54±8** | |
| **XVi** | **50±8** | | **55±7** | |

Thus, practically all benzimidazole derivatives significantly prolonged the life of Drosophila, often 3-4 times. The most effective geroprotective compounds are the following substances: IIIh; IXa; IXb; XIa; XIIIh; XVa; XVg; XVh; XVi, which extended the life of fruit flies up to 50 days or more, while in the control this indicator was 21-26 days, with the expected value of 30 days. The most effective was the compound XVg, which extended the lifespan of Drosophila by almost 3 times to 70 days and was a trimethyl derivative of benzimidazolyl acridoneacetic acid.

## Claims

**1.** Derivative of benzimidazole of formula I

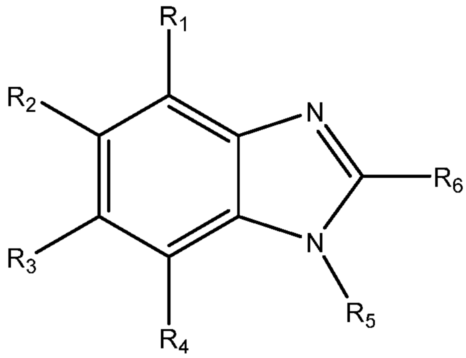

where $R_{1-4}$ = H, $-CH_3$, $-C_2H_5$, -Br, - Cl, -I, -F, -OH, -CN, $-NO_2$, -COOH.
$R_5$= H, $-CH_3$, $-C_2H_5$.

$R_6$=

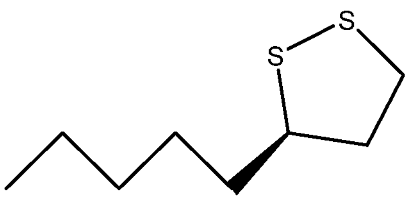

**2.** Derivatives according to claim 1, wherein

$R_6$= 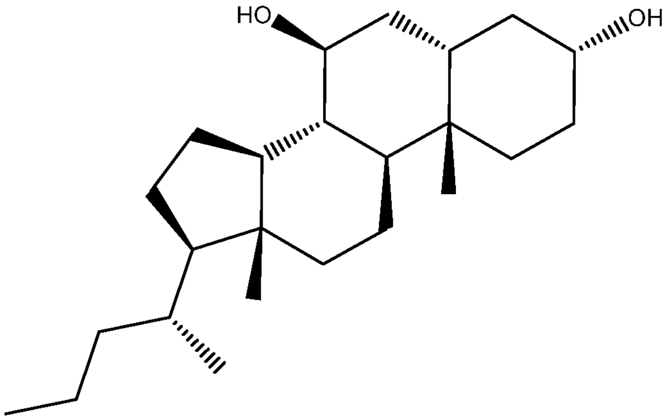

3. Derivatives according to claim 1, wherein

$R_6$= 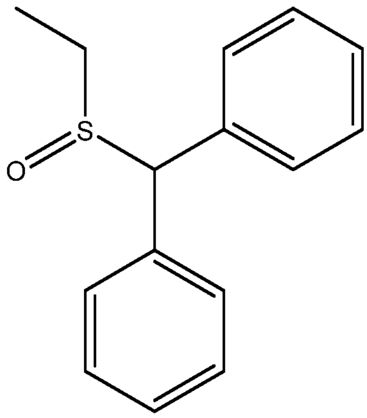

4. Derivatives according to claim 1, wherein

$R_6$= 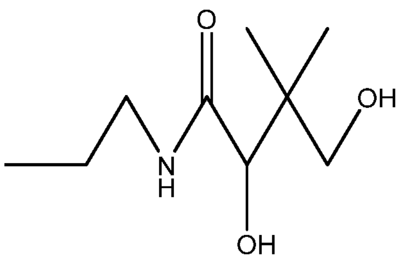

5. Derivatives according to claim 1, wherein

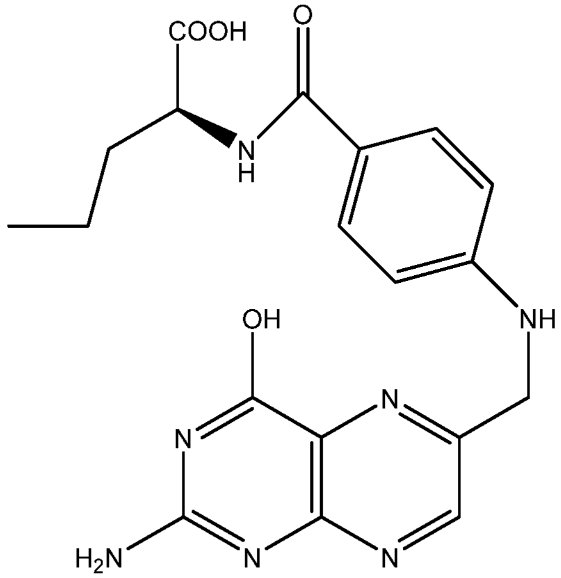

R6=

6. Derivatives according to claim 1, wherein

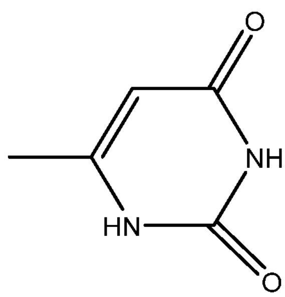

R6=

7. Derivatives according to claim 1, wherein

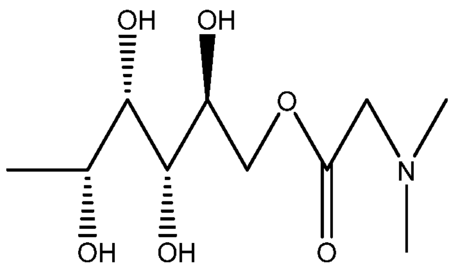

R6=

8. Derivatives according to claim 1, wherein

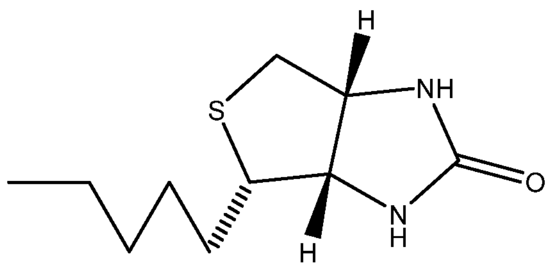

R6=

9. Derivatives according to claim 1, wherein

$R_6$= 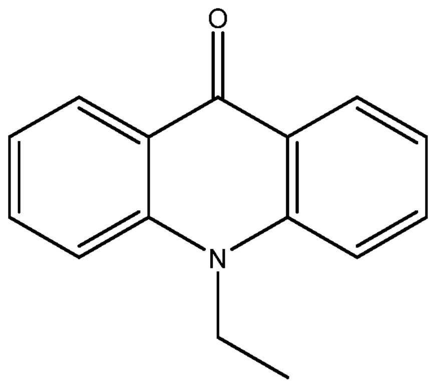

**10.** Derivatives according to claim 1, wherein

$R_6$= 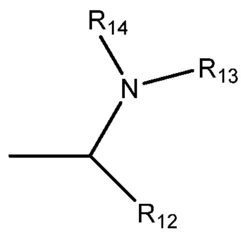

$R_{13,14}$= -H; - $CH_3$, -$C_2H_5$
$R_{12}$= - $CH_3$

**11.** Derivatives according to claim 10, wherein

$R_{12}$= 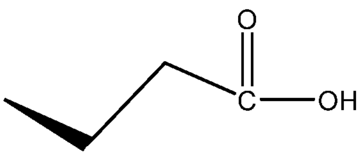 ;

**12.** Derivatives according to claim 10, wherein

$R_{12}$= 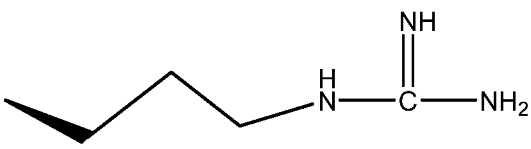

**13.** Derivatives according to claim 10, wherein

$R_{12}$= 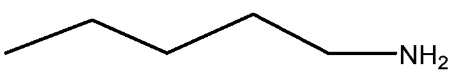

**14.** Derivatives according to claim 10, wherein

$R_{12}$= 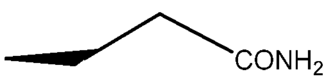

**15.** Derivatives according to claim 10, wherein

$R_{12}$= 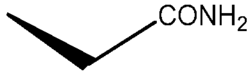

**16.** Derivatives according to claim 10, wherein

$R_{12}$= 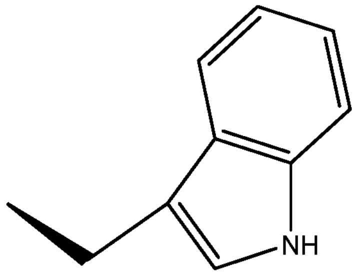

**17.** Derivatives according to claim 10, wherein

$R_{12}$= 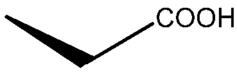

**18.** Derivatives according to claims 1 to 17, **wherein** they are salts: hydrochlorides, hydrobromides, hydroiodides, sodium, potassium, magnesium, calcium, iron, copper salts.

**19.** Derivatives according to cl. 1 -17, **wherein** they are salts with thioctic acid.

**20.** Derivatives according to cl. 1-17, **wherein** they are salts with metformin.

**21.** Derivatives according to claims 1 to 17, **wherein** they are salts with choline.

**22.** Derivatives according to cl. 1 -17, **wherein** they are a complex with dapagliflozin.

**23.** Derivatives according to cl. 1-17., **wherein** they represent a complex with 3- (1H-benzimidazol-2-yl) -1,2,2-trimethylcyclopentanecarboxylic acid.

**24.** The use of derivatives according to paragraphs. 1-17 in the manufacture of medicines - selective activators of imidazoline receptors for the treatment of pathologies caused by aging of the body.

**25.** A pharmaceutical composition for the treatment of pathologies caused by aging of the body, containing an effective amount of derivatives according to claims 1 to 24 from 0.5 mg to 1000 mg in combination with one or more pharmaceutically acceptable excipients.

**26.** A pharmaceutical composition according to claim 25, **wherein** the presence of thioctic acid in its composition.

**27.** A pharmaceutical composition according to claim 25, **wherein** metformin in its composition also presented.

**28.** A pharmaceutical composition according to claim 25, **wherein** in its composition is also choline presented.

**29.** A pharmaceutical composition according to claim 25, **wherein** in its composition also is dapagliflozin presented.

**30.** A pharmaceutical composition according to claim 25, **wherein** in its composition of 3- (1H-benzimidazol-2-yl) -1,2,2-trimethylcyclopentanecarboxylic acid.

**31.** The pharmaceutical composition according to claim 25, **wherein** in its composition also of a mixture of dapagliflozin, choline, metformin, thioctic acid, 3- (1H-benzimidazol-2-yl) -1,2,2-trimethylcyclopentanecarboxylic acid.

**32.** A pharmaceutical composition according to claim 25, **wherein** it presented in the form of injection solutions or suspensions, or multi-dose vials, in the form of simple or coated tablets, sugar-coated tablets, plate capsules, gel capsules, pills, wafers, powders, suppositories or rectal capsules, solutions or suspensions, ointments and gels for topical use.

**33.** A pharmaceutical composition according to claim 25, **wherein** it suitable for parenteral, oral, rectal, permucous or transdermal administration.

**34.** The use of the pharmaceutical composition according to claim 25, **wherein** drug for the treatment of pathologies caused by aging of the body is anticancer agents.

**INTERNATIONAL SEARCH REPORT**

International application No.
PCT/RU 2020/000019

A. CLASSIFICATION OF SUBJECT MATTER
See the supplemental sheet

According to International Patent Classification (IPC) or to both national classification and IPC

B. FIELDS SEARCHED

Minimum documentation searched (classification system followed by classification symbols)
C07D 235/06, C07D 403/06, C07D 409/06, C07D 471/04, C07D 495/04, C07J 43/00, A61K 31/4184, A61P 35/00, A61P 43/00

Documentation searched other than minimum documentation to the extent that such documents are included in the fields searched

Electronic data base consulted during the international search (name of data base and, where practicable, search terms used)
EAPATIS, ESPACENET, PatSearch (RUPTO internal), Information Retrieval System of FIPS, USPTO, PATENTSCOPE, STNext, PubMed, E-Library, Google

C. DOCUMENTS CONSIDERED TO BE RELEVANT

| Category* | Citation of document, with indication, where appropriate, of the relevant passages | Relevant to claim No. |
|---|---|---|
| X<br>A | WO 2004/078172 A2 (YISSUM RESEARCH DEVELOPMENT COMPANY OF THE HEBREW UNIVERSITY OF JERUSALEM et al.) 16.09.2004, claim 27, p. 8, compound 7, claim 32 | 1, 18, 19<br>2-17, 20-34 |
| X<br>A | MOREAU Robert J. et al. «Fragment-Based Drug Discovery of Inhibitors of Pho sphopantetheine Adenylyltransferase from Gram-Negative Bacteria». Journal of Medicinal Chemistry, 2018, 61 (8), c. 3309-3324 (1-52), doi:10.1021/acs.jmedchem.7b01691, abstract, table 1, plan 5, compound 29 | 4<br>1-3, 5-34 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 August 2020 (17.08.2020) | 15 October 2020 (15.10.2020) |
| Name and mailing address of the ISA/<br>RU<br><br>Facsimile No. | Authorized officer<br><br><br>Telephone No. |

Form PCT/ISA/210 (second sheet) (April 2005)

**INTERNATIONAL SEARCH REPORT**

International application No.
PCT/RU 2020/000019

C (Continuation). DOCUMENTS CONSIDERED TO BE RELEVANT

| Category* | Citation of document, with indication, where appropriate, of the relevant passages | Relevant to claim No. |
|---|---|---|
| X | ROEDER Carl N. et al. «Benzimidazole studies. I. The mechanism of benzimidazole formation from o -phenylenedi amine» . The Journal of Organic Chemistry, 1941, 06(1), c. 25-35, doi: 10.1021/jo01201a002, p. 29, second paragraph from below, p. 31, lines 3-7, p. 30, fist plan, section «Eksperimentalnaia chast», compound II, III, X, ikh gidrokhloridnye soli | 10, 18 |
| A | | 1-9, 11-17, 19-34 |
| X | WO 2004/056784 A1 (BOEHRINGER INGELHEIM PHARMA GMBH & SO. KG et al.) 08.07.2004, abstract, section «Otsenka», examples 8, 12, 56 | 10 |
| A | | 1-9, 11-34 |
| X<br>A | PubChem CID: 3698260, 10-( 1 H-Benzimidazol-2-ylmethyl) acridin-9-one, 10.09.2005 [on-line data bases] [retrieved on 17.08.2020] | 9<br>1-8, 10-34 |

Form PCT/ISA/210 (continuation of second sheet) (April 2005)

INTERNATIONAL SEARCH REPORT

International application No.
PCT/RU 2020/000019

CLASSIFICATION OF SUBJECT MATTER

***C07D 235/06*** *(2006.01)*
***C07D 403/06*** *(2006.01)*
***C07D 409/06*** *(2006.01)*
***C07D 471/04*** *(2006.01)*
***C07D 495/04*** *(2006.01)*
***C07J 43/00*** *(2006.01)*
***A61K 31/4184*** *(2006.01)*
***A61P 35/00*** *(2006.01)*
***A61P 43/00*** *(2006.01)*

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2000002878 A1


### Non-patent literature cited in the description


- **HAENNI, A. ; LITHELL, H.** Treatment with a β-blocker with β2-agonism improves glucose and lipid metabolism in essential hypertension. *Metabolism,* 1994, vol. 43 (4), 455-461
- **KRESPI PG ; MAKRIS TK ; HATZIZACHARIAS AN et al.** Moxonidine effect on microalbuminuria, thrombomodulin, and plasminogen activator inhibitor - 1 levels in patients with essential hypertension. *J Cardiovasc Drugs and Therapy,* 1998, vol. 12, 463-467
- 2007 Guidelines for the Management of Arterial Hypertension: The Task Force for the Management of Arterial Hypertension of the European Society of Hypertension (ESH) and of the European Society of Cardiology (ESC). *J Hypertens,* 2007, vol. 25 (6), 1105-87